# EUROPEAN PATENT APPLICATION

(11) **EP 3 949 870 A1**
(43) Date of publication of application: **09.02.2022**
(21) Application number: 21189228.6
(22) Date of filing: 02.08.2021
(51) Int. Cl.: A61B 17/072

(54) **SURGICAL STAPLING DEVICE WITH STAPLE CARTRIDGE HAVING DUMMY PORTION**

(30) Priority: 03.08.2020 US 202016983169
(71) Applicant: Covidien LP, Mansfield, MA 02048 (US)
(72) Inventor: GEORGE, Sabastian Koduthully, 562125 Bangalore (IN); SHANMUGAVEL, Logamurugaraj, 642007 Pollachi (IN)
(74) Representative: Maschio, Antonio

(57) **Abstract**

A surgical stapling device includes a channel member and a staple cartridge that is received within the channel member and is formed from a functional portion and a dummy portion that are coupled together. The functional portion of the staple cartridge defines a knife slot and a plurality of staple receiving pockets. Each of the staple receiving pockets supports a staple and a pusher that is movable within the respective staple receiving pocket to eject the staple from within the staple receiving pocket. The dummy portion of the staple cartridge defines a knife slot and is devoid of staples.

## Description

### FIELD

This disclosure is directed to a surgical stapling device including a staple cartridge, and more particularly, to a surgical stapling device including a staple cartridge having a functional portion and a dummy portion.

### BACKGROUND

Surgical stapling devices typically include a tool assembly having anvil assembly and a cartridge assembly that are movable in relation to each other between open and clamped positions. The cartridge assembly includes a staple cartridge and a channel. The staple cartridge is removably supported within the channel to allow replacement of the staple cartridge after use to facilitate reuse of the surgical stapling device. Staple cartridges are available in a variety of lengths including 30mm, 45mm, and 60mm. The tool assembly includes a drive member including a knife bar that is movable along the length of the staple cartridge to fire rows of staples from the staple cartridge and to cut tissue between the rows of staples.

In certain surgical procedures, e.g., sleeve gastrectomy procedures, the length of the staple cartridge is longer than the tissue that must be stapled and cut such that a distal portion of the staple cartridge is not engaged with tissue to be treated. As such, when the stapling device is fired to eject the rows of staples from the staple cartridge, the staples ejected from the distal portion of the staple cartridge are ejected into a body cavity of the patient.

A continuing need exists in the stapling arts for a staple cartridge that is adapted to minimize waste.

### SUMMARY

One aspect of this disclosure is directed to a cartridge assembly including a channel member and a staple cartridge. The channel member defines a cavity. The staple cartridge includes a proximal portion and a distal portion. The proximal portion includes a body defining a first knife slot and a first plurality of staple receiving pockets and includes a plurality of staples and pushers. One staple of the plurality of staples is received within each pocket of the first plurality of staple receiving pockets. The pushers are movable within body of the proximal portion from a first position to a second position to eject the plurality of staples from within the first plurality of staple receiving pockets. The distal portion of the staple cartridge includes a body defining a second knife slot and is devoid of staples. The proximal portion is adapted to be coupled to the distal portion such that the first and second knife slots are in axial alignment.

Another aspect of this disclosure is directed to a surgical stapling device including an elongate body, and a tool assembly. The elongate body has a proximal portion and a distal portion. The tool assembly is supported on the distal portion of the elongate body and includes an anvil and a cartridge assembly coupled to the anvil such that the tool assembly is movable between open and clamped positions. The cartridge assembly includes a channel member and a staple cartridge. The channel member defines a cavity. The staple cartridge includes a proximal portion and a distal portion. The proximal portion includes a body defining a first knife slot and a first plurality of staple receiving pockets and includes a plurality of staples and pushers. One staple of the plurality of staples is received within each pocket of the first plurality of staple receiving pockets. The pushers are movable within body of the proximal portion from a first position to a second position to eject the plurality of staples from within the first plurality of staple receiving pockets. The distal portion of the staple cartridge includes a body defining a second knife slot and is devoid of staples. The proximal portion is adapted to be coupled to the distal portion such that the first and second knife slots are in axial alignment.

Another aspect of this disclosure is directed to a kit including a staple guard and a staple cartridge. The staple guard defines a channel having a proximal portion and a distal portion. The staple cartridge includes a proximal portion, a first distal portion, and a second distal portion. The proximal portion of the staple cartridge is retained within the proximal portion of the channel of the staple guard. The proximal portion of the staple cartridge includes a body defining a first knife slot and a plurality of staple receiving pockets and including a plurality of staples and pushers. Each staple of the plurality of staples of the proximal portion of the staple cartridge is received within one pocket of the plurality of staple receiving pockets of the proximal portion of the staple cartridge. The first distal portion of the staple cartridge is configured to be received within the distal portion of the channel of the staple guard. The first distal portion of the staple cartridge includes a body defining a knife slot and a plurality of staple receiving pockets and including a plurality of staples and pushers. Each staple of the plurality of staples of the first distal portion of the staple cartridge is received within one pocket of the plurality of staple receiving pockets of the first distal portion of the staple cartridge. The second distal portion of the staple cartridge is configured to be received within the distal portion of the staple guard and defines a central knife slot. The second distal portion of the staple cartridge is devoid of staples.

In aspects of the disclosure, the body of the proximal portion of the staple cartridge has a distal end that defines recesses and the body of the distal portion of the staple cartridge has a proximal end that defines extensions.

In some aspects of the disclosure, the extensions are received within the recesses to couple the proximal portion of the staple cartridge to the distal portion of the staple cartridge.

In certain aspects of the disclosure, the staple cartridge includes a staple guard that defines a channel that receives the proximal and distal portions of the staple cartridge.

In aspects of the disclosure, the proximal portion of the staple cartridge is fixedly secured within the channel of the staple guard.

In some aspects of the disclosure, the distal portion of the staple cartridge is removably received within the channel of the staple guard.

In certain aspects of the disclosure, the distal portion of the staple cartridge defines a plurality of staple receiving pockets.

In aspects of the disclosure, the cartridge assembly includes an actuation sled.

Other features of the disclosure will be appreciated from the following description.

### BRIEF DESCRIPTION OF THE DRAWINGS

Various aspects of the disclosed surgical stapling device are described herein below with reference to the drawings, wherein:
FIG. 1 is a side perspective view of a surgical stapling device including aspects of the disclosure with a tool assembly in an open position;
FIG. 2 is an enlarged view of the indicated area of detail shown in FIG. 1;
FIG. 3 is an exploded view of a cartridge assembly of the tool assembly of the stapling device shown in FIG. 1;
FIG. 4 is a side perspective view of a staple cartridge of the cartridge assembly shown in FIG. 3 assembled;
FIG. 5 is an enlarged view of the indicated area of detail shown in FIG. 4;
FIG. 6 is a side perspective view of the tool assembly of the stapling device shown in FIG. 2 with the tool assembly in a clamped position about tissue after the tool assembly is fired;
FIG. 7 is a side perspective view of the of the stapled and cut tissue after the tissue has been stapled and cut with the tool assembly shown in FIG. 6; and
FIG. 8 is a kit including the staple cartridge shown in FIG. 4.

### DETAILED DESCRIPTION

The disclosed surgical stapling device including a staple cartridge according to aspects of the disclosure will now be described in detail with reference to the drawings in which like reference numerals designate identical or corresponding elements in each of the several views. However, it is to be understood that the disclosed aspects are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through a small diameter incision or cannula. Further, the term "clinician" is used generally to refer to medical personnel including doctors, nurses, and support personnel. In addition, directional terms such as front, rear, upper, lower, top, bottom, distal, proximal, and similar terms are used to assist in understanding the description and are not intended to limit the disclosure.

The disclosed surgical stapling device includes a channel member and a staple cartridge that is received within the channel member. The staple cartridge is formed from a functional portion and a dummy portion that are coupled together. The functional portion of the staple cartridge includes a body that defines a knife slot and a plurality of staple receiving pockets. Each of the staple receiving pockets supports a staple and a pusher that is movable within the respective staple receiving pocket to eject the staple from the staple receiving pocket. The dummy portion of the staple cartridge defines a knife slot but does not include staples.

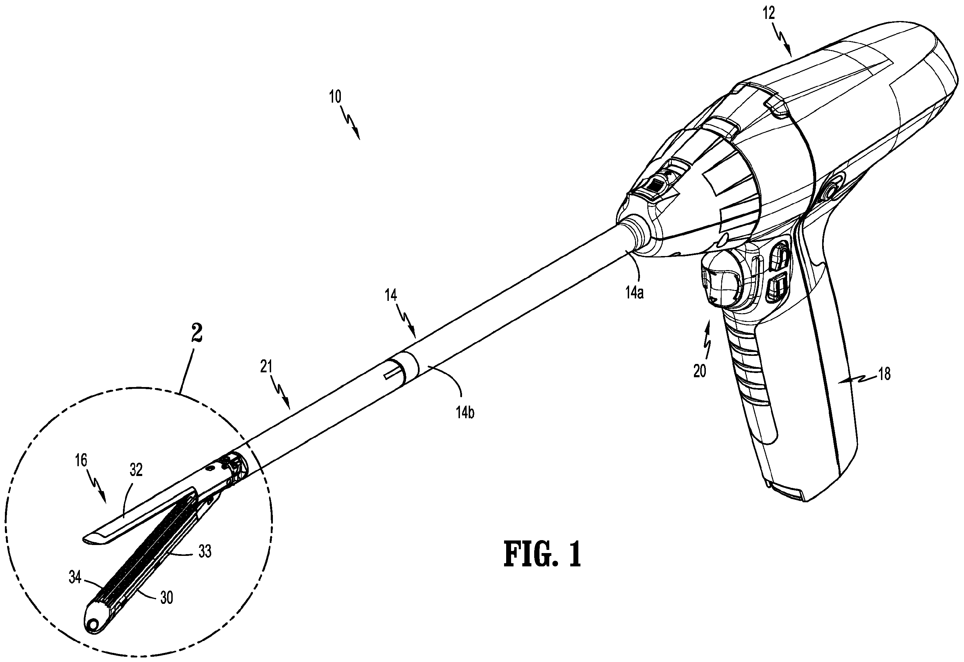

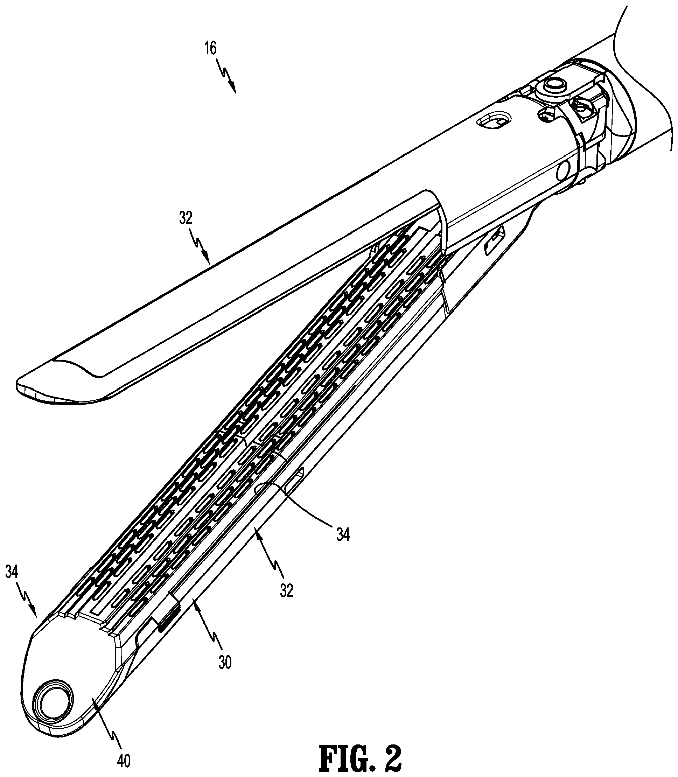

FIGS. 1 and 2 illustrate a surgical stapling device shown generally as stapling device 10 (FIG. 1) that includes a handle assembly 12, an elongate body or adapter assembly 14, and a tool assembly 16. As illustrated, the handle assembly 12 is powered and includes a stationary handgrip 18 and actuation buttons 20. The actuation buttons 20 are operable to actuate various functions of the tool assembly 16 via the adapter assembly 14 including approximation, stapling, and dissection. In certain aspects of the disclosure, the handle assembly 16 supports batteries (not shown) that provide power to the handle assembly 12 to operate the stapling device 10. Although the stapling device 10 is illustrated as a powered stapling device, it is envisioned that the disclosed tool assembly 16 is suitable for use with manually powered surgical stapling devices as well as robotically controlled stapling devices.

The adapter assembly 14 includes a proximal portion 14a and a distal portion 14b. The proximal portion 14a is coupled to the handle assembly 12 and the distal portion 14b supports the tool assembly 16. In aspects of the disclosure, the tool assembly 16 forms part of a reload assembly 21 that is removably supported on the distal portion 14b of the adapter assembly 14 and can be replaced after the stapling device 10 is fired to facilitate reuse of the stapling device 10. It is also envisioned that the tool assembly 16 can be fixedly coupled to the distal portion of the adapter assembly 14.

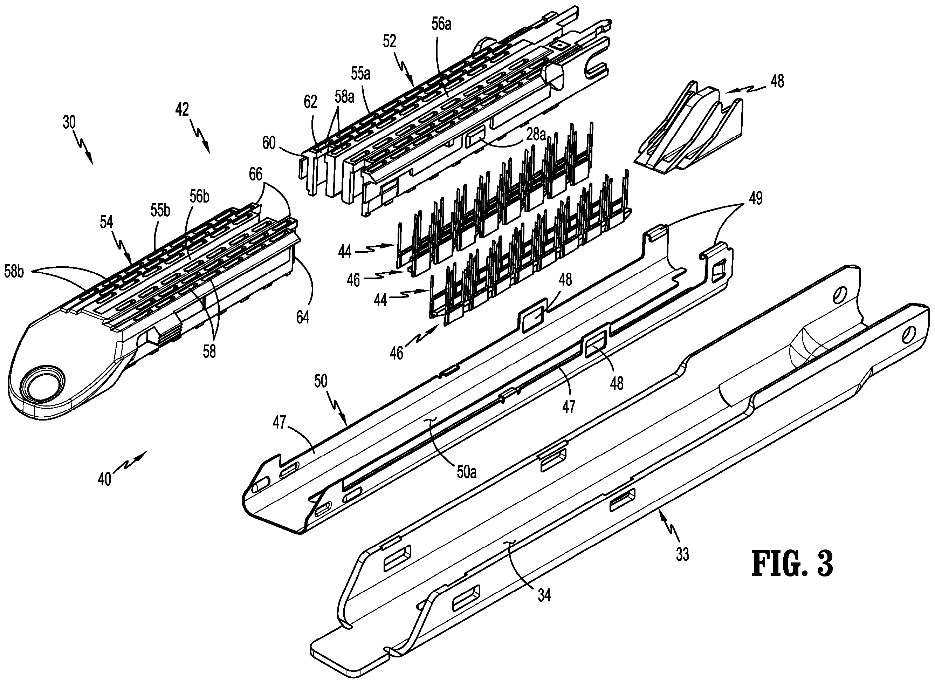

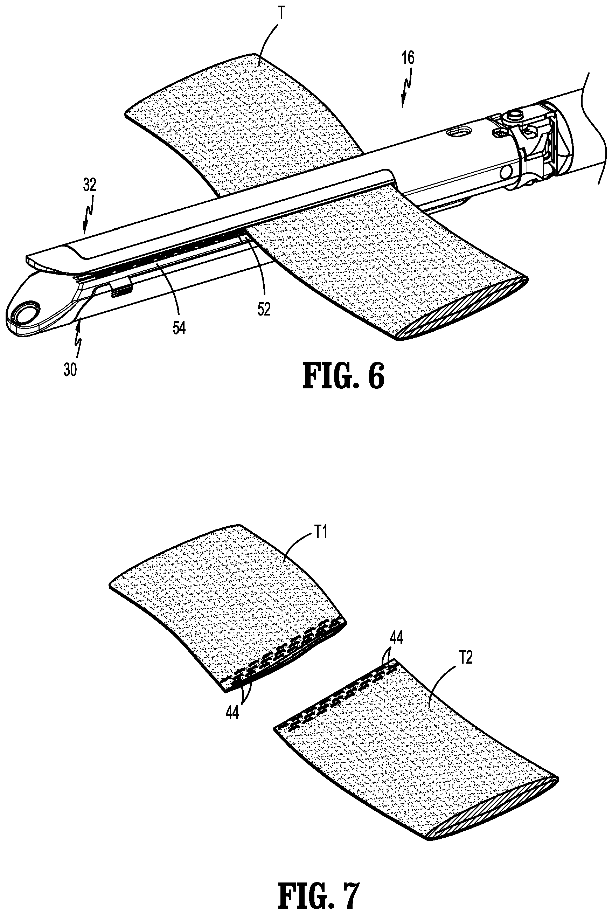

The tool assembly 16 of the stapling device 10 includes a cartridge assembly 30 and an anvil 32. The cartridge assembly 30 and the anvil 32 are coupled together such that the tool assembly 16 can pivot between an open position (FIG. 1) and a clamped position (FIG. 6). In the clamped position, the anvil 32 and the cartridge assembly 30 are in close juxtaposed alignment with each other. The cartridge assembly 30 includes a channel member 33 that defines a cavity 34 (FIG. 3) and a staple cartridge 40 that is received in the cavity 34. In aspects of the disclosure, the staple cartridge 40 can be removably received within the cavity 34 of the channel member 33 to facilitate replacement of the staple cartridge 40 after each firing of the stapling device 10.

FIGS. 3-5 illustrate the staple cartridge 40 which includes a cartridge body 42, a plurality of staples 44 (FIG. 3), pushers 46 (FIG. 3), an actuation sled 48, and a staple guard 50. The cartridge body 42 of the staple cartridge 40 is received within the cavity 34 defined by the channel member 33 and includes a proximal portion 52 and a distal portion 54 that is separate from the proximal portion 52. In aspects of the disclosure, each of the proximal and distal portions 52 and 54 includes a tissue engaging surface 55a and 55b, respectively, that define a central knife slot 56a and 56b, respectively, and a plurality of staple receiving pockets 58a and 58b, respectively. Each of the staple receiving pockets 58a in the proximal portion 52 of the cartridge body 42 receives a staple 44 (FIG. 3) and at least a portion of one of the pushers 46 (FIG. 3). The actuation sled 48 is movable from a proximal end of the cartridge body 42 distally into sequential engagement with the pushers 46 to urge the pushers 46 upwardly as viewed in FIG. 3 to eject the staples 44 from the staple receiving pockets 58a of the proximal portion 52 of the cartridge body 42. U.S. Patent No. 6,241,139 discloses exemplary aspects of the construction and operation of a staple cartridge of a surgical stapling device.

The staple guard 50 defines a channel 50a (FIG. 3) that receives the cartridge body 42. The staple guard 50 is secured to a lower portion of the proximal and distal portions 52 and 54 of the cartridge body 42 to retain the staples 44 and the pushers 46 within the proximal portion 52 of the cartridge body 42. In aspects of the disclosure, the staple guard 50 includes side walls 47 (FIG. 3) that include openings 48 and fingers 49. The openings 48 receive protrusions 48a formed on the proximal portion 52 of the cartridge body 42 and the fingers 49 engage an upper surface of the proximal portion 52 of the cartridge body 42 to retain the proximal portion 52 of the cartridge body 42 within channel 50a of the staple guard 50. It is envisioned that the proximal portion of the cartridge body 42 can be secured within the staple guard 50 using adhesive or welding.

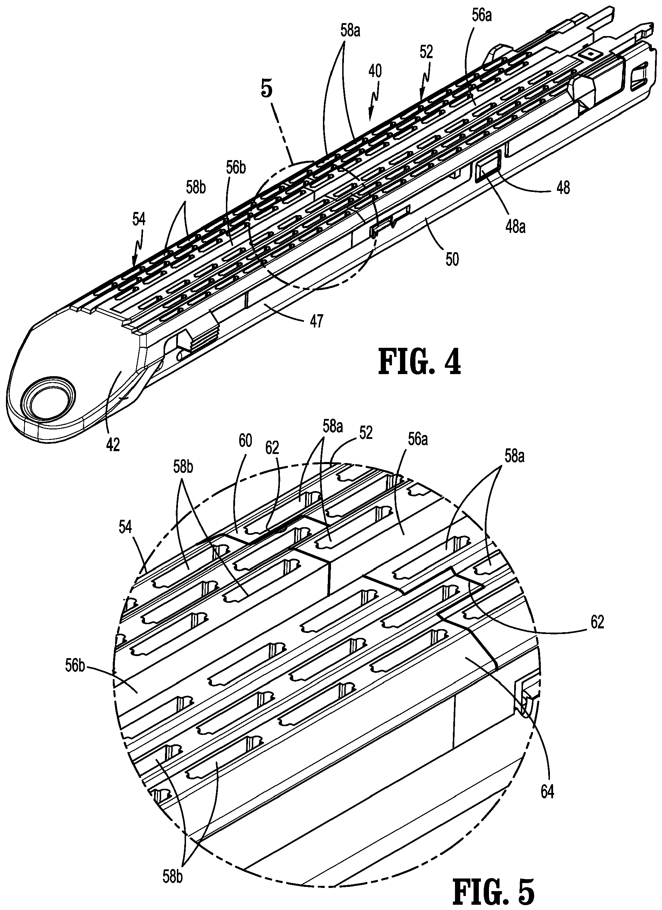

The proximal and distal portions 52 and 54 of the cartridge body 42 are coupled together and received within the channel 50a of the staple guard 50. In aspects of the disclosure, the proximal portion 52 of the cartridge body 42 has a distal end 60 (FIG. 3) and defines two spaced, proximally extending longitudinal recesses 62 (FIG. 3). One of the longitudinal recesses 62 is positioned on each side of the central knife slot 56a. The distal portion 54 of the cartridge body 42 includes a proximal end 64 and two longitudinal extensions 66 that extend proximally from the proximal end 64 and are received within the longitudinal recesses 62 of the proximal portion 52 when the distal portion 54 of the cartridge body 42 is coupled to the proximal portion 54 of the cartridge body 42. When the distal portion 54 of the cartridge body 42 is coupled to the proximal portion 52 of the cartridge body 42 (FIG. 5), the tissue engaging surfaces 55a and 55b of the proximal and distal portions 52 and 54 of the cartridge body 42 are contiguous with each other and the proximal end 60 of the proximal portion 52 of the cartridge body 42 is in abutting relation to the proximal end 64 of the distal portion of the cartridge body 42.

In aspects of the disclosure, the distal portion 54 of the cartridge body 42 can be a functional portion of the staple cartridge 40 or a non-functional portion or dummy portion of the staple cartridge 40. Where the distal portion 54 of the cartridge body 42 is a functional portion of the staple cartridge 40, the distal portion 54 includes staples 44 and pushers 46 and functions in the same manner as the proximal portion of the staple cartridge 40. Where the distal portion 54 of the cartridge body 42 is a dummy portion, the distal portion 54 does not include staples 44 or pushers 46. It is envisioned that where the distal portion 54 of the cartridge body 42 is a dummy portion, the distal portion 54 need not define staple receiving pockets 58 or include pushers 46. However, in order to facilitate passage of a knife bar (not shown) through the staple cartridge 40, the dummy portion includes a knife slot 56.

FIGS. 6 and 7 illustrate the tool assembly 16 of the stapling device 10 (FIG. 1) with the tool assembly 16 clamped about tissue "T" such as during a sleeve gastrectomy procedure. The cartridge body 42 of the staple cartridge 40 includes a proximal portion 52 that is functional (includes staples 44 and pushers 46) and a distal portion 54 of the cartridge body 42 that is a dummy portion that does not include staples 44 or pushers 46. When the stapling device 10 is fired, the staples 44 from the proximal portion 52 of the cartridge body 42 are formed in the tissue "T" as a knife blade (not shown) moves through the tissue "T" to divide the tissue "T" into first and second tissue segments "Tl" and "T2" (FIG. 7).

It is envisioned that the distal portion 54 of the body 42 (FIG. 3) can also be functional, i.e., include staples 44 and pushers 46. In such a stapling device 10, when the stapling device is fired, the stapling device 10 will form staples along the length of the body 42 of the staple cartridge 40.

Although illustrated as being of substantially equal length, it is envisioned that the proximal portion 52 of the cartridge body 42 and the distal portion 54 of the cartridge body 42 can be of different lengths. For example, the proximal portion 52 of the cartridge body 42 can be 15mm, 30mm, 45mm, 60mm, or any other length that is suitable to perform a surgical procedure and the distal portion can be 15mm, 30mm, 45mm or any other length suitable to perform the surgical procedure.

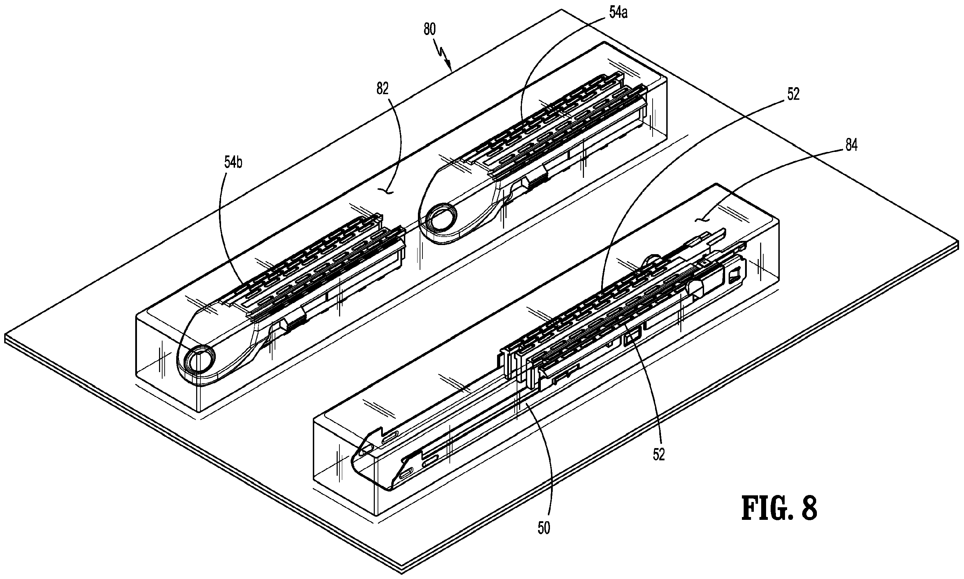

FIG. 8 illustrates a kit for performing a surgical procedure, e.g., a sleeve gastrectomy procedure, including a staple cartridge 40 that includes a staple guard 50, and a cartridge body 42 that includes a proximal portion 52 and two distal portions 54a and 54b. The proximal portion 52 is functional and includes staples 44 and pushers 46 (FIG. 3). The distal portion 54a is a dummy portion and (although may include staple pockets), does not include staples or pushers. The distal portion 54b of the cartridge body 42 is functional and includes staples 44 (FIG. 3) and pushers 46 (FIG. 3). During a surgical procedure, such as a sleeve gastrectomy procedure, where the staple cartridge 40 is longer than the tissue to be treated, the distal portion 54a can be secured to the proximal portion 52 within the staple guard 50. During a surgical procedure where the staple cartridge 40 is of a length that corresponds to the length of the tissue to be treated, the distal portion 54b can be secured to the proximal portion 52 within the staple guard 50 to treat the tissue.

In aspects of the disclosure, the staple cartridge 40 can be received within a package 80 (FIG. 8). In certain aspects of the disclosure, the package 80 can include two pockets 82 and 84. The pocket 82 can receive an assembly 86 that includes the staple guard 50 and the proximal portion 52 of the staple cartridge 40. In some aspects of the disclosure, the proximal portion 52 can be fixedly secured to the staple guard 50. The second pocket 84 can include the distal portions 54a and 54b of the cartridge body 40.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A cartridge assembly comprising:
   a channel member defining a cavity;
   a staple cartridge including a proximal portion and a distal portion, the proximal portion including a body defining a first knife slot and a first plurality of staple receiving pockets and including a plurality of staples and pushers, one staple of the plurality of staples is received within each pocket of the first plurality of staple receiving pockets, the pushers movable within body of the proximal portion from a first position to a second position to eject the plurality of staples from within the first plurality of staple receiving pockets, the distal portion of the staple cartridge including a body defining a second knife slot, the distal portion of the staple cartridge being devoid of staples, wherein the proximal portion is adapted to be coupled to the distal portion such that the first and second knife slots are in axial alignment.
2. The cartridge assembly of paragraph 1, wherein the body of the proximal portion of the staple cartridge has a distal end that defines recesses and the body of the distal portion of the staple cartridge has a proximal end that defines extensions, the extensions received within the recesses to couple the proximal portion of the staple cartridge to the distal portion of the staple cartridge.
3. The cartridge assembly of paragraph 2, wherein the staple cartridge includes a staple guard, the staple guard defining a channel, the channel receiving the proximal and distal portions of the staple cartridge.
4. The cartridge assembly of paragraph 3, wherein the proximal portion of the staple cartridge is fixedly secured within the channel of the staple guard.
5. The cartridge assembly of paragraph 4, wherein the distal portion of the staple cartridge is removably received within the channel of the staple guard.
6. The cartridge assembly of paragraph 1, wherein the distal portion of the staple cartridge defines a plurality of staple receiving pockets.
7. The cartridge assembly of paragraph 1, wherein the cartridge assembly includes an actuation sled.
8. A surgical stapling device comprising:
   an elongate body having a proximal portion and a distal portion;
   a tool assembly supported on the distal portion of the elongate body, the tool assembly including an anvil and a cartridge assembly coupled to the anvil such that the tool assembly is movable between open and clamped positions, the cartridge assembly including
      a proximal portion and a distal portion, the proximal portion including a body defining a first knife slot and a first plurality of staple receiving pockets and including a plurality of staples and pushers, one staple of the plurality of staples received within each of the staple receiving pockets of the body of the proximal portion, the pushers movable within the proximal portion from a first position to a second position to eject the plurality of staples from within the first plurality of staple receiving pockets, the distal portion including a body defining a second knife slot, the distal portion of the staple cartridge being devoid of staples;
   wherein the proximal portion is adapted to be coupled to the distal portion such that the first and second knife slots are in axial alignment.
9. The surgical stapling device of paragraph 8, wherein the body of the proximal portion of the staple cartridge has a distal end that defines recesses and the body of the distal portion of the staple cartridge has a proximal end that defines extensions, the extensions received within the recesses to couple the proximal portion of the staple cartridge to the distal portion of the staple cartridge.
10. The surgical stapling device of paragraph 9, wherein the staple cartridge includes a staple guard, the staple guard defining a channel, the channel receiving the proximal and distal portions of the staple cartridge.
11. The surgical stapling device of paragraph 10, wherein the proximal portion of the staple cartridge is fixedly secured within the channel of the staple guard.
12. The surgical stapling device of paragraph 11, wherein the distal portion of the staple cartridge is removably received within the channel of the staple guard.
13. The surgical stapling device of paragraph 8, wherein the distal portion of the staple cartridge defines a plurality of staple receiving pockets.
14. A kit comprising:
   a staple guard defining a channel having a proximal portion and a distal portion;
   a staple cartridge including a proximal portion, a first distal portion, and a second distal portion, the proximal portion of the staple cartridge retained within the proximal portion of the channel of the staple guard, the proximal portion of the staple cartridge including a body defining a first knife slot and a plurality of staple receiving pockets and including a plurality of staples and pushers, each staple of the plurality of staples of the proximal portion of the staple cartridge received within one pocket of the plurality of staple receiving pockets of the proximal portion of the staple cartridge, the first distal portion of the staple cartridge configured to be received within the distal portion of the channel of the staple guard, the first distal portion of the staple cartridge including a body defining a knife slot and a plurality of staple receiving pockets and including a plurality of staples and pushers, each staple of the plurality of staples of the first distal portion of the staple cartridge received within one second staple receiving pockets of the plurality of second staple receiving pockets of the first distal portion of the staple cartridge, the second distal portion of the staple cartridge configured to be received within the distal portion of the staple guard and defining a central knife slot, the second distal portion of the staple cartridge being devoid of staples.
15. The kit of paragraph 14, wherein the body of the proximal portion of the staple cartridge has a distal end that defines recesses and the first and second distal portions of the body of the staple cartridge each have a proximal end that defines extensions, the extensions of one of the first or second distal portions adapted to be received within the recesses of the proximal portion of the staple cartridge to couple the proximal portion of the body of the staple cartridge to one of the first or second distal portions of the staple cartridge.
16. The kit of paragraph 14, wherein the second distal portion of the staple cartridge defines a plurality of staple receiving pockets.

## Claims

1. A cartridge assembly comprising:
a channel member defining a cavity;
a staple cartridge including a proximal portion and a distal portion, the proximal portion including a body defining a first knife slot and a first plurality of staple receiving pockets and including a plurality of staples and pushers, one staple of the plurality of staples is received within each pocket of the first plurality of staple receiving pockets, the pushers movable within body of the proximal portion from a first position to a second position to eject the plurality of staples from within the first plurality of staple receiving pockets, the distal portion of the staple cartridge including a body defining a second knife slot, the distal portion of the staple cartridge being devoid of staples, wherein the proximal portion is adapted to be coupled to the distal portion such that the first and second knife slots are in axial alignment.

2. The cartridge assembly of claim 1, wherein the body of the proximal portion of the staple cartridge has a distal end that defines recesses and the body of the distal portion of the staple cartridge has a proximal end that defines extensions, the extensions received within the recesses to couple the proximal portion of the staple cartridge to the distal portion of the staple cartridge.

3. The cartridge assembly of claim 2, wherein the staple cartridge includes a staple guard, the staple guard defining a channel, the channel receiving the proximal and distal portions of the staple cartridge.

4. The cartridge assembly of claim 3, wherein the proximal portion of the staple cartridge is fixedly secured within the channel of the staple guard.

5. The cartridge assembly of claim 4, wherein the distal portion of the staple cartridge is removably received within the channel of the staple guard.

6. The cartridge assembly of any preceding claim, wherein the distal portion of the staple cartridge defines a plurality of staple receiving pockets.

7. The cartridge assembly of any preceding claim, wherein the cartridge assembly includes an actuation sled.

8. A surgical stapling device comprising:
an elongate body having a proximal portion and a distal portion;
a tool assembly supported on the distal portion of the elongate body, the tool assembly including an anvil and a cartridge assembly coupled to the anvil such that the tool assembly is movable between open and clamped positions, the cartridge assembly including
a proximal portion and a distal portion, the proximal portion including a body defining a first knife slot and a first plurality of staple receiving pockets and including a plurality of staples and pushers, one staple of the plurality of staples received within each of the staple receiving pockets of the body of the proximal portion, the pushers movable within the proximal portion from a first position to a second position to eject the plurality of staples from within the first plurality of staple receiving pockets, the distal portion including a body defining a second knife slot, the distal portion of the staple cartridge being devoid of staples;
wherein the proximal portion is adapted to be coupled to the distal portion such that the first and second knife slots are in axial alignment.

9. The surgical stapling device of claim 8, wherein the body of the proximal portion of the staple cartridge has a distal end that defines recesses and the body of the distal portion of the staple cartridge has a proximal end that defines extensions, the extensions received within the recesses to couple the proximal portion of the staple cartridge to the distal portion of the staple cartridge.

10. The surgical stapling device of claim 9, wherein the staple cartridge includes a staple guard, the staple guard defining a channel, the channel receiving the proximal and distal portions of the staple cartridge.

11. The surgical stapling device of claim 10, wherein the proximal portion of the staple cartridge is fixedly secured within the channel of the staple guard.

12. The surgical stapling device of claim 11, wherein the distal portion of the staple cartridge is removably received within the channel of the staple guard.

13. The surgical stapling device of any of claims 8 to 12, wherein the distal portion of the staple cartridge defines a plurality of staple receiving pockets.

14. A kit comprising:
a staple guard defining a channel having a proximal portion and a distal portion;
a staple cartridge including a proximal portion, a first distal portion, and a second distal portion, the proximal portion of the staple cartridge retained within the proximal portion of the channel of the staple guard, the proximal portion of the staple cartridge including a body defining a first knife slot and a plurality of staple receiving pockets and including a plurality of staples and pushers, each staple of the plurality of staples of the proximal portion of the staple cartridge received within one pocket of the plurality of staple receiving pockets of the proximal portion of the staple cartridge, the first distal portion of the staple cartridge configured to be received within the distal portion of the channel of the staple guard, the first distal portion of the staple cartridge including a body defining a knife slot and a plurality of staple receiving pockets and including a plurality of staples and pushers, each staple of the plurality of staples of the first distal portion of the staple cartridge received within one second staple receiving pockets of the plurality of second staple receiving pockets of the first distal portion of the staple cartridge, the second distal portion of the staple cartridge configured to be received within the distal portion of the staple guard and defining a central knife slot, the second distal portion of the staple cartridge being devoid of staples.

15. The kit of claim 14, wherein the body of the proximal portion of the staple cartridge has a distal end that defines recesses and the first and second distal portions of the body of the staple cartridge each have a proximal end that defines extensions, the extensions of one of the first or second distal portions adapted to be received within the recesses of the proximal portion of the staple cartridge to couple the proximal portion of the body of the staple cartridge to one of the first or second distal portions of the staple cartridge; preferably wherein the second distal portion of the staple cartridge defines a plurality of staple receiving pockets.
